# EUROPEAN PATENT SPECIFICATION

(11) **EP 1 848 356 B1**
(45) Date of publication and mention of the grant of the patent: **05.11.2008**
(21) Application number: 06709773.3
(22) Date of filing: 16.02.2006
(51) Int. Cl.: A61B 18/00, F17C 7/04, A61B 18/14, A61B 18/18, A61B 18/12

(54) **TISSUE TREATMENT SYSTEM**
GEWEBEBEHANDLUNGSSYSTEM
SYSTEME DE TRAITEMENT DE TISSU

(30) Priority: 17.02.2005 GB 0503382
(43) Date of publication of application: 31.10.2007
(73) Proprietor: RHYTEC LIMITED, Hungerford, Berkshire RG17 0UN (GB)
(72) Inventor: GOBLE, Nigel Mark, Newbury, Berkshire RG20 0HX (GB); PENNY, Keith, Monmouth NP25 5FD (GB); FERNIE, Douglas, Stapleford, Cambridge CB2 5DX (GB); WARD, Robert M., Oakington, Cambridge CB4 5AQ (GB); VARNEY, Kelvin J., Abergavenny, Monmouthshire NP7 8SU (GB); JENKINS, Andrew, Pentre, Mid Glamorgan CF41 7AW (GB)
(74) Representative: Blatchford, William Michael
(86) International application number: PCT/GB2006/000536
(87) International publication number: WO 2006/087547

(56) References cited:
- US-A1- 2002 161 362
- US-A1- 2003 069 576
- US-A1- 2003 125 727
- US-B1- 6 226 996
- US-B1- 6 475 215

## Description

This invention relates to a tissue treatment system including a radio frequency (r.f.) generator and a treatment instrument connectible to the generator and to a source of ionisable gas for producing a plasma jet. The primary use of the system is skin resurfacing.

A tissue-treatment system is disclosed in U.S. Patents Nos. 6,629,974 filed February 13, 2002 and 6,723,091 filed February 22, 2001, and U.S. Patent Application Serial No. 10/792,765 filed March 5, 2004. In this known system, a handheld treatment instrument has a gas conduit terminating in a plasma exit nozzle. There is an electrode associated with the conduit, and this electrode is coupled to a separate r.f. power generator which is arranged to deliver r.f. power to the electrode for creating a plasma from gas fed through the conduit. The delivered radio frequency power is typically at UHF frequencies in the region of 2.45GHz and the instrument includes a structure resonant in that frequency region in order to provide an electric field concentration in the conduit for striking the plasma upstream of the exit nozzle, the plasma forming a jet which emerges from the nozzle and which can be used to effect local heating of a tissue surface.

U.S. Patent No. 6,226,996 discloses a device for cooling a surface, such as human skin, to a desired temperature. The device comprise a reservoir for a cryogenic fluid having a valve to controllably release the fluid from the reservoir, and means to form a mist of the fluid released from the reservoir and to direct the mist at the surface to cool an area. A non-contact temperature sensor measures the temperature of the surface within the cooled area, and a control unit connected to the temperature sensor activates the temperature sensor. The control unit can be programmed by a user with the desired final temperature, and an optical source connected to the control unit produces a beam of light that illuminates at least a point in the cooled area and that can be used for positioning the device.

The applicants have found that the clinical effect caused through energy delivered by the pulsed plasma instrument is, for a given instrument design and generator energy setting, dependent, firstly, on the distance of the plasma exit nozzle from the tissue to be treated; as the distance increases the plasma jet beam or plume becomes more diffuse, causing the energy and the energy per unit area to decrease, so reducing the heating effect. Secondly, the clinical effect is dependent on the angle of the direction of the plasma jet (in the known system the jet is coaxial with the longitudinal axis of the instrument) with respect to the tissue to be treated. It will be appreciated that, as the angle becomes more extreme then, for an otherwise circular distribution of energy, the distribution becomes elliptical or oval with differing concentration of energy per unit area at either end of the long axis of the oval.

It is desirable to indicate to the user the area of the tissue that will be heated to produce desirable clinical effects so that the user may accurately target areas of tissue. More accurate overlapping of adjacent treatments can be achieved, giving an overall uniform delivery of energy to the patient's tissue to be treated.

According to a first aspect of the present invention, a tissue treatment system includes a treatment instrument in the form of a handpiece which is arranged to direct a beam of treatment energy from a distal end of the handpiece along a treatment beam axis for treating a tissue surface spaced from the said distal end, the treatment energy being produced by a treatment energy emitter, wherein the handpiece incorporates at least part of an optical target marking projector for projecting a visible target marker, wherein the target marking projector has a light exit aperture at the distal end of the handpiece, the light exit aperture being offset from the treatment beam axis, and wherein the projector has a projection axis which is inclined towards the treatment beam axis to intersect the latter substantially at a predetermined spacing from the handpiece distal end, the projector being arranged to project the marker onto a plane at the predetermined spacing. The invention has particular application to a tissue treatment system which operates by delivering thermal energy to a tissue surface such as human skin. Such thermal energy treatment may be performed by a treatment energy emitter in the form of a gas plasma generator, the above-mentioned handpiece having a nozzle at its distal end for directing a thermal energy beam in the form of a plasma jet outwardly from the nozzle.

Advantageously, the target marking projector is arranged to project a diverging, e.g. substantially conical, light beam from the exit aperture. Thus, the size of the projected marker on a tissue surface varies according to the distance of the handpiece from the tissue.

In the preferred system, the projector comprises a light source and an optical fibres light guide having an entrance end and an exit end. At least a distal portion of the light guide is housed in the handpiece and the light source is arranged such that at least a component of light generated by the source and incident on the entrance end of the light guide is incident at an angle to the central axis of the light guide. The diverging beam may be generated by causing the light source to apply a converging beam to the entrance end of the light guide.

The projected target marker preferably defines an area indicative of the tissue area which will be subjected to thermal treatment when the distal end of the handpiece is located at the predetermined spacing from a tissue surface. Thus, for instance, a circular treatment area may be indicated by a target marker in the form of a generally circular ring of projected light. Other markers can be used, such as a plurality of light dots arranged in a suitable pattern, a square, parts of a circle, and so on.

The projector typically comprises a light source and an optical fibre light guide, at least a distal portion of the light guide being housed in the handpiece and terminating in the exit aperture.

The light source itself may be formed in the shape of the required marker and may be an illuminated mask having a marker aperture of the required shape. Thus, for a ring marker, the mask may have an annular aperture or an aperture forming a part or parts of an annulus. Alternatively, the light source may be a light emitter which is, itself, in the form of an annulus.

The projector preferably includes at least one lens for concentrating light from the light source onto a proximal end of the fibre guide, light reaching the end of the fibre guide at an angle within the acceptance angle for the material of the fibre or fibres. Under such conditions, light incident at a given angle on the proximal end of the fibre is emitted from the distal end at the same angle with respect to the fibre axis. This property of optical fibres may, therefore, be used to form an image of the light source in the projection plane.

Although the projector may be battery-powered and may be contained entirely within the handpiece, the preferred embodiment has the light source in the same housing as the treatment energy power source (which may be a radio frequency generator housing) and light radiation is transmitted via an optical fibre contained within a cord connecting the handpiece to the power source housing. In the case of a gas plasma system this cord also contains a coaxial cable and a gas supply tube.

Projecting a target marker as described onto the tissue surface indicates the area on the tissue surface that will be treated. The size of the marker indicates the spacing of the handpiece from the tissue surface since, with diverging light emitted from the exit aperture, the size of the marker increases as the distance of the tissue surface from the handpiece increases. In general terms, because the size of the marker varies as the distance varies, the marker size indicates the distance.

The shape of the marker indicates the angle at which the instrument is held with respect to the target tissue surface. Thus, if the light source is circular, a circular marker image indicates that the treatment beam axis is approximately perpendicular to the target tissue surface. If the marker image is elliptical, then this is an indication of an inclined target beam axis.

According to a second aspect of the invention, a tissue treatment instrument for a tissue treatment system comprises a handpiece which is arranged to direct a beam of treatment energy from a distal end thereof along a treatment beam axis defined by the handpiece for treating a tissue surface spaced from the said distal end, wherein the handpiece incorporates optical means for projecting a visible target marker, and wherein the optical means has a light exit aperture which is offset from the treatment beam axis and defines a projection axis inclined with respect to the treatment beam axis to intersect the latter substantially at a predetermined spacing from the handpiece distal end, the optical means being arranged to project the marker onto a plane at the predetermined spacing.

Human skin has two principal layers: the epidermis, which is the outer layer and typically has a thickness of around 120µ in the region of the face, and the dermis which is typically 20-30 times thicker than the epidermis, and contains hair follicles, sebaceous glands, nerve endings and fine blood capillaries. By volume the dermis is made up predominantly of the protein collagen.

Ageing and exposure to ultraviolet (UV) light result in changes to the structure of the skin, these changes including a loss of elasticity, sagging, wrinkling and a pallor or yellowing of the skin consistent with reduced vascularity. The changes produce fine lines or wrinkles in the structure of the skin tissue. The background to these effects is explained in our co-pending international patent application entitled "Cosmetic Method of Regenerating the Reticular Architecture of the Dermis", claiming priority from British Patent Application No. 0503330.3, the disclosure of which is incorporated herein by reference.

Described hereinafter is a cosmetic method of regenerating the reticular architecture of skin tissue that has degenerated to form a line or wrinkle in the tissue using a handheld tissue treatment instrument as a source of thermal energy, wherein the method comprises locating the instrument over the tissue to be treated whilst illuminating the surface of the tissue with a visible target marker projected from the instrument, the position of the instrument with respect to the tissue surface being selected according to the appearance of the marker, and operating the thermal energy source whilst the instrument is in the said position.

Preferably it is the spacing of the instrument from the tissue surface which is selected according to the appearance of the marker. In addition, the angle of the instrument with respect to the tissue surface may be selected in this way.

The instrument nozzle may be used as a size reference feature, the method including comparing the marker size with the nozzle diameter to achieve optimum instrument spacing. The selected spacing of the nozzle from the tissue surface is preferably in the range of from 2mm to 10mm and, most normally, in the range of from 4mm to 7mm.

The thermal energy source is operated for a single pass over the skin surface, the thermal energy source being arranged to have an energy setting dependent on the desired depth of effect. Alternatively, the thermal energy source is operated over at least two passes over the skin surface, the energy levels of the passes being chosen dependent on the desired depth of effect.

In either case, the energy setting of the thermal energy source may be such as to create vacuolation on the first pass. In the latter case, the energy setting of the thermal energy source may be such as not to create vacuolation on the first pass, thereby enabling a second pass without removing the treated skin.

Preferably, the energy setting of the thermal energy source is such as to preserve the integrity of the epidermis as a biological dressing.

The thermal energy source is operated so that a line of cleavage occurs within the skin two to five days following treatment, the line of cleavage occurring between said first and second regions. In one particular case, the operation of the thermal energy source may be such as to form a line of cleavage from two to three cells deep in the stratum corneum of the superficial epidermis and the upper dermis.

Advantageously, the operation of the thermal energy source is such that the tissue in the first region is sloughed tissue. In this case, the sloughed tissue is removed once a new epidermis has been substantially generated in the region of the line of cleavage.

Preferably, the tissue below the line of cleavage in said second region includes the lower epidermis, the basal membrane and the DE Junction. More preferably, at least the thermally-modified basal membrane and the DE Junction are regenerated.

In one particular case, the line of cleavage forms below areas of solar elastosis, such that the solar elastosis and deranged fibroblasts are sloughed.

Preferably, the operation of the thermal energy source is such as to denature dermal collagen in the second region.

The tissue in said second region undergoes a regenerative process following regeneration of the epidermis.

In this case, the reticular architecture of the dermis is regenerated in whole, or in part, by fibroblasts less exposed to the effects of UV radiation.

The collagen architecture and/or elastin architecture and/or the GAGS of the dermis is regenerated in whole, or in part, by fibroblasts less exposed to the effects of UV radiation.

Preferably, the healing process is such that risk of scarring and hypo pigmentation is substantially eliminated.

Advantageously, a progressive improvement in skin changes associated with ageing and photodamage occur over a period of between six and twelve months following treatment.

The source of thermal energy is an instrument having an electrode connected to a power output device, and wherein the power output device is operated to create an electric field in the region of the electrode; a flow of gas is directed through the electric field to generate, by virtue of the interaction of the electric field with the gas, a plasma; the plasma is directed onto the tissue for a predetermined period of time; and the power transferred into the plasma from the electric field is controlled so as to desiccate at least a portion of the dermis with vapour pockets formed in dermis cells.

Preferably, the power output device is operated to deliver discrete pulses of heat of millisecond duration.

Advantageously, the pulses have a duration in the range of from about 0.5 milliseconds or 2 milliseconds to about 100 milliseconds, and preferably a duration in the range of from about 4.5 to about 15.4 milliseconds.

Conveniently, the power output device is operated to deliver energy in the range of from about 1 Joule to about 4 Joules for an instrument having a first predetermined nozzle diameter, and to deliver energy in the range of from less than 0.5 Joules to about 2.0 Joules for an instrument having a second predetermined diameter that is less than the first predetermined diameter.

Preferably, the first predetermined diameter is substantially 5mm and the second predetermined diameter is substantially 1.5mm.

The thermal energy may be delivered to the tissue from a thermal energy source as a jet of fluid having stored heat energy at the tissue surface, the jet of fluid typically comprising a jet of ionised diatomic gas.

There is also disclosed a cosmetic method of regenerating the reticular architecture of the dermis using a tissue treatment system including a treatment instrument in the form of a handpiece having a gas plasma generator, wherein the method comprises locating the instrument over the tissue to be treated adjacent to a line or wrinkle projecting a visible marker from the instrument onto the tissue surface beneath the instrument, positioning the instrument to cause the marker to adopt a required configuration associated with predetermine position of the instrument with respect to the tissue surface, and operating the gas plasma generator whilst the instrument is in the predetermined position to direct a gas plasma jet onto the tissue surface.

There is also disclosed a cosmetic method of regenerating the reticular architecture of tissue using a handheld tissue treatment instrument as a source of thermal energy comprises locating the instrument over the tissue to be treated adjacent a line or wrinkle, illuminating the surface of the tissue with a visible target marker projected from the instrument, and using the marker as a positioning aid. Typically, this method includes operating the thermal energy source with the instrument located at a plurality of different positions to produce a graduated clinical effect at the periphery of a treated tissue area. The instrument positions may be selected by locating the instrument so as to produce different respective marker configurations on the tissue surface.

The gradation of effect may be produced by positioning the instrument at different spacings or at different angles with respect to the tissue surface. Thus, the gradation of effect may be produced by increasing the spacing on each successive pass of the instrument from treated to untreated areas of tissue, using the projected marker as a spacing guide or by angling the instrument at a greater angle in respect of the perpendicular at the boundaries of a treated area, the angle being selected by observing the shape of the marker. In the case of a marker which is generally circular when the instrument is perpendicular to the tissue surface, the instrument may be increasingly inclined by selecting orientations which produce a marker shape which is increasingly elliptical.

Said method is not a therapeutic method, it being a cosmetic method that may be carried out to improve the appearance of the skin in regions adjacent to lines or wrinkles in the structure of skin tissue.

This method of applying thermal energy to the surface of the skin in selected regions (adjacent to lines or wrinkles in skin tissue) produces an improved appearance of such regions, and in particular reduces the depth of fine lines and wrinkles produced by ageing and sun exposure. Such regions include any area that has been exposed to the sun; but, more particularly, involves skin regions normally exposed during day-to-day activities, such as the face, the neck and the hands, the aim of the cosmetic method being to produce a more youthful appearance.

When applying the method to the face, there are particular areas where wrinkling of the skin (particularly amongst the female population) is more common and pronounced. These includes areas around the eyes and the upper lip, which do not respond well to other commonly-used types of cosmetic procedures, such as the injection of Botulinum Toxin to paralyse subcutaneaous muscles. The current invention is particularly useful in producing cosmetic outcomes in these areas.

When applying the thermal energy, a selected area (adjacent to a line or wrinkle in the skin tissue) is treated with one pass of energy by applying consecutive pulses, each of which produces a circular effect, typically of the order of 9-10mm in diameter with a 10-20% overlap to the skin surface. To further improve the cosmetic result, a second pass may be performed, whereby the skin to be treated is stretched, such that the valley and sides of the wrinkle are exposed, and pulses of energy are applied along the line of the wrinkle. By this means, the edges of the wrinkle valley are exposed to a higher aggregate intensity of thermal energy, such that the depth of the line of the cleavage produced by the method is deeper, and the wrinkle is blended in with the relative height of adjacent un-wrinkled skin.

The longevity of the cosmetic outcome is then produced by regeneration of the reticular architecture below the skin surface that in effect fixes the change in contour of the wrinkle, reducing the propensity for it to reform to the same degree in association with changes for example, in facial expression. Once again, due to the method of application, this fixation is greatest at the edges of the valley of the wrinkle which contract inwards in association with the regenerative process.

The cosmetic effect of the method is, therefore, distinguishable from a therapeutic effect in that it is carried out only in regions of skin tissue adjacent to lines or wrinkles in that tissue. Another distinction between said cosmetic method and a therapeutic method is that, at most, two passes over the skin surface are made as mentioned above, the second pass (where used) being effective to stretch the skin so that the valley and sides of the wrinkle are subjected to a greater depth of treatment. This is to be contrasted with therapeutic treatments, where the use of multiple passes is commonplace. For example, multiple passes are essential for many therapeutic methods, such as treating lesions with the wiping away of treated tissue between passes (this being akin to a progressive shaving away of a lesion).

The invention will now be described by way of example with reference to the drawings in which:
Figure 1 is a diagrammatic view of a tissue treatment system in accordance with the invention;
Figure 2 is a longitudinal cross-section of a tissue treatment instrument forming part of the system of Figure 1;
Figure 3 is a block diagram of a radio frequency generator for use in the system of Figure 1;
Figure 4 is a diagram showing an optical target marking projector of the system of Figure 1;
Figure 5 is an exploded perspective view of the tissue treatment instrument shown in Figure 2;
Figure 6 is a cross-section of a light source forming part of the target marking projector of Figure 4;
Figure 7 is an axial view of a light source mask;
Figure 8 is a diagram showing the principle of the transmission of a target marker image in an optical fibre;
Figure 9 is a detail from Figure 4 showing the distal end of the treatment instrument and the projection of the marker image onto a tissue surface;
Figure 10 is a composite diagram showing the regeneration of the reticular architecture of the dermis when using the system of Figures 1 to 9 for different pulse widths and energy settings; and
Figures 11 to 13 show the process of reticular regeneration at the day of treatment, at four days after treatment, and at ten days after treatment respectively.

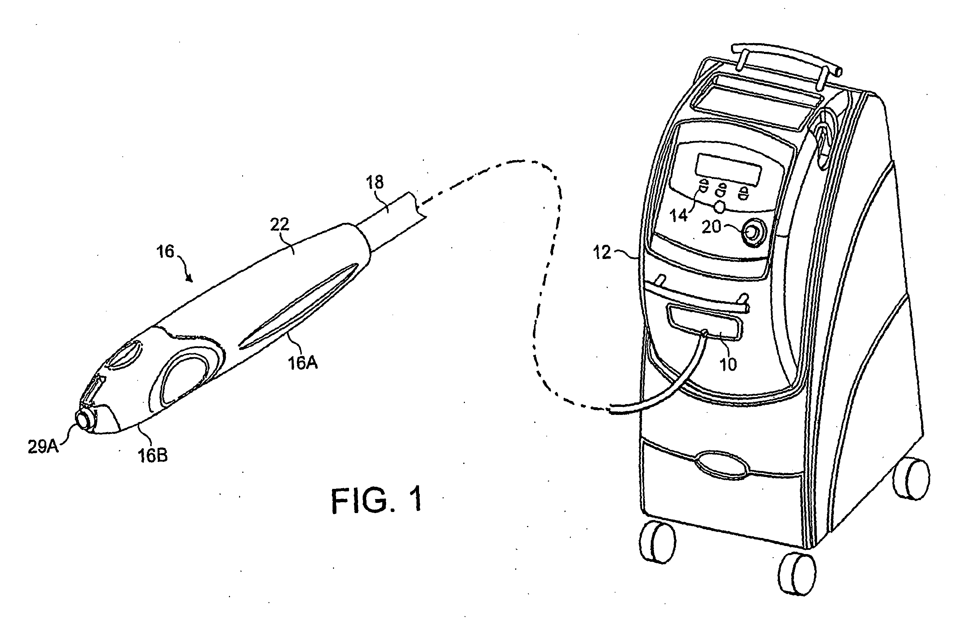

Referring to Figure 1, a tissue treatment system in accordance with the invention has a treatment power source in the form of an r.f. generator 10 mounted in a floor-standing generator housing 12 and having a user interface 14 for setting the generator to different energy level settings. A handheld tissue treatment instrument 16 is connected to the generator by means of a cord 18. The instrument 16 comprises a handpiece having a re-usable handpiece body 16A and a disposable nose assembly 16B.

The generator housing 12 has an instrument holder 20 for storing the instrument when not in use.

Within the cord 18 there is a coaxial cable for conveying r.f. energy from the generator 10 to the instrument 16, and a gas supply pipe for supplying nitrogen gas from a gas reservoir or source (not shown) inside the generator housing 12. The cord also contains an optical fibre line for transmitting visible light to the instrument from a light source in the generator housing. At its distal end, the cord 18 passes into the casing 22 of the handpiece body 16A

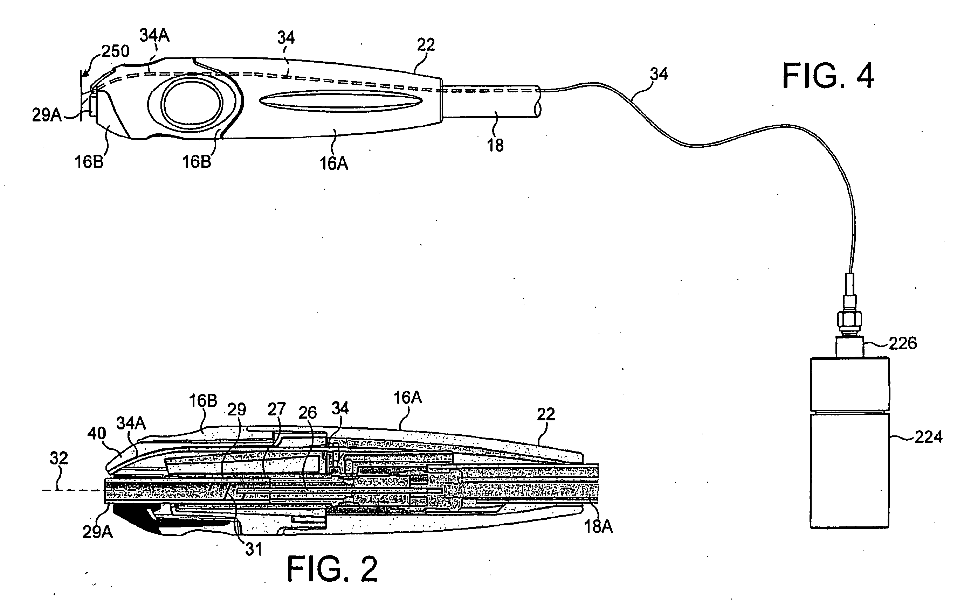

In the re-usable handpiece body 16A, the coaxial cable 18A is connected to inner and outer electrodes 26 and 27, as shown in Figure 2, thereby coupling the electrodes to the generator to receive r.f. power. The inner electrode 26 extends longitudinally within the outer electrode 27. Between them is a gas conduit in the form of a heat-resistant tube 29 (preferably made of quartz) housed in the disposable instrument nose assembly 16B. When the nose assembly 16B is secured to the handpiece body 16A, the interior of the tube 29 is in communication with the gas supply pipe interior, the nose assembly 16B being received within the body 16A such that the inner and outer electrodes 26, 27 are associated with the tube, the inner electrode 26 extending axially into the tube 29 and the outer electrode 27 extending around the outside of the tube 29.

A resonator in the form of a helically wound tungsten coil 31 is located within the quartz tube 29, the coil being positioned such that, when the disposable nose assembly 16B is secured in position on the handpiece body 16A, the proximal end of the coil is adjacent the distal end of the inner electrode 26. The coil is wound such that it is adjacent and in intimate contact with the inner surface of the quartz tube 29.

In use of the instrument, nitrogen gas is fed by a supply pipe to the interior of the tube 29 where it reaches a location adjacent the distal end of the inner electrode 26. When an r.f. voltage is supplied via the coaxial cable to the electrodes 26 and 27, an intense r.f. electric field is created inside the tube 29 in the region of the distal end of the inner electrode. The field strength is aided by the helical coil 31 which is resonant at the operating frequency of the generator and, in this way, conversion of the nitrogen gas into a plasma is promoted, the plasma exiting as a j et at a nozzle 29A of the quartz tube 29. The plasma jet, centred on a treatment beam axis 32 (this axis being the axis of the tube 29), is directed onto tissue to be treated, the nozzle 29A typically being held a few millimetres from the surface of the tissue.

The handpiece 16 also contains an optical fibre light guide 34 which extends through the core 18 into the handpiece where its distal end portion 34A is bent inwardly towards the treatment axis defined by the quartz tube 29 to terminate at a distal end which defines an exit aperture adjacent the nozzle 29A. The inclination of the fibre guide at this point defines a projection axis for projecting a target marker onto the tissue surface, as will be described in more detail below.

Following repeated use of the instrument, the quartz tube 29 and its resonant coil 31 require replacement. The disposable nose assembly 16B containing these elements is easily attached and detached from the reusable part 16A of the instrument, the interface between the two components 16A, 16B of the instrument providing accurate location of the quartz tube 29 and the coil 31 with respect to the electrodes 26, 27.

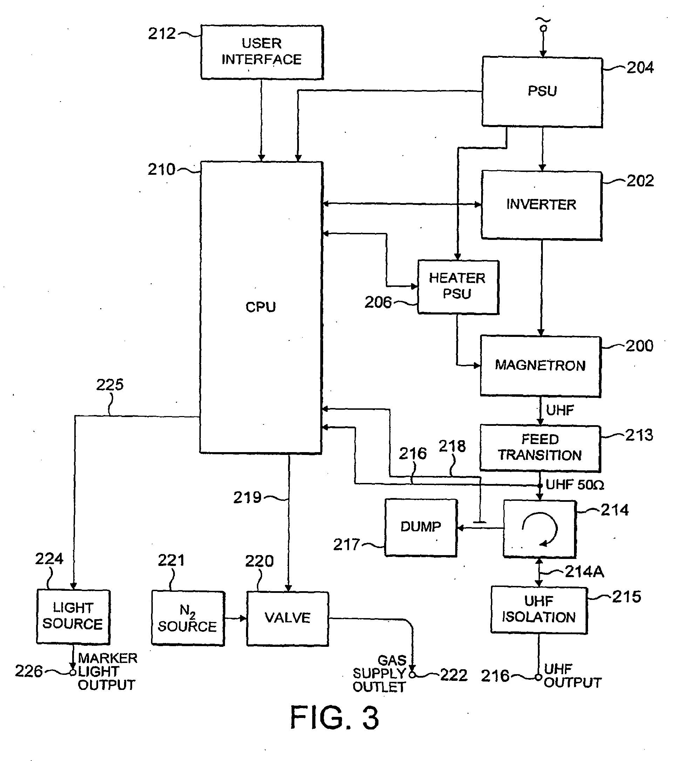

Referring to Figure 3, r.f. energy is generated in a magnetron 200. Power for the magnetron 200 is supplied in two ways, firstly as a high DC voltage for the cathode, generated by an inverter 202 supplied from a power supply unit 204 and, secondly, as a filament supply for the cathode heater from a heater power supply unit 206. Both the high voltage supply represented by the inverter 202 and the filament supply 206 are coupled to a CPU controller 210 for controlling the power output of the magnetron. A user interface 212 is coupled to the controller 210 for the purpose of setting the power output mode, amongst other functions.

The magnetron 200 operates in the high UHF band, typically at 2.475GHz, producing an output on an output line which feeds a feed transition stage 213 for converting the magnetron output to a coaxial 50 ohms feeder, low frequency AC isolation also being provided by this stage. Thereafter, a circulator 214 provides a constant 50 ohms load impedance for the output of the feed transition stage 213. Apart from a first port coupled to the transition stage 213, the circulator 214 has a second port 214A coupled to a UHF isolation stage 215 and hence to the output terminal 216 of the generator for delivering RF power to the handheld instrument 16 (Figure 1). Reflected power is fed from the circulator 214 to a resistive power dump 215. Forward and reflected power sensing connections 216 and 218 provide sensing signals for the controller 210.

The controller 210 also applies via line 219 a control signal for opening and closing a gas supply valve 220 so that nitrogen gas is supplied from the source 221 to a gas supply outlet 222 from where it is fed through the gas supply pipe in the cord 18 to the instrument 16 (Figure 1), when required. A light source 224, forming part of the above-mentioned optical target marker projector, is connected to the controller 210 by a control line 225 and produces a target marker light beam at an optical marker light output 226.

The controller 210 is programmed to pulse the magnetron 200 so that, when the user presses a footswitch (not shown in the drawings), r.f. energy is delivered as a pulsed waveform to the UHF output 216, typically at a pulse repetition rate of between about 1Hz and about 4Hz. A single pulse mode is also provided. The pulses preferably have durations in the range of from 2ms to 100ms. The controller 210 also operates the valve 220 so that nitrogen gas is supplied to the handheld instrument simultaneously with the supply of r.f. energy. The light source 224 can be actuated independently of r.f. energy and nitrogen gas supply. Further details of the modes of delivery of r.f. energy are set out in the above-mentioned U.S. Patent No. 6,723,091.

The optical fibre light guide 34 and the light source 224 form part of an optical target marker projector which is shown as a whole in Figure 4. The light source 224 is in the generator housing 12 (see Figure 1) and, coupled to its optical output 226, is an optical fibre line 34 which passes through the cord 18 connecting the handpiece 16 to the generator housing 12 and, thence, into the casing 22 of the handpiece. Within the handpiece, the fibre guide 34 extends generally parallel to and offset from the treatment beam axis 32 until it reaches a distal end portion of the handpiece. There, the distal end portion 34A of the fibre guide is bent towards the treatment beam axis 32, as shown. The distal end of the fibre guide 34 forms an exit aperture for the guided marker beam which, when the light source 224 is activated, is projected as a diverging beam onto the tissue surface 250 to be treated.

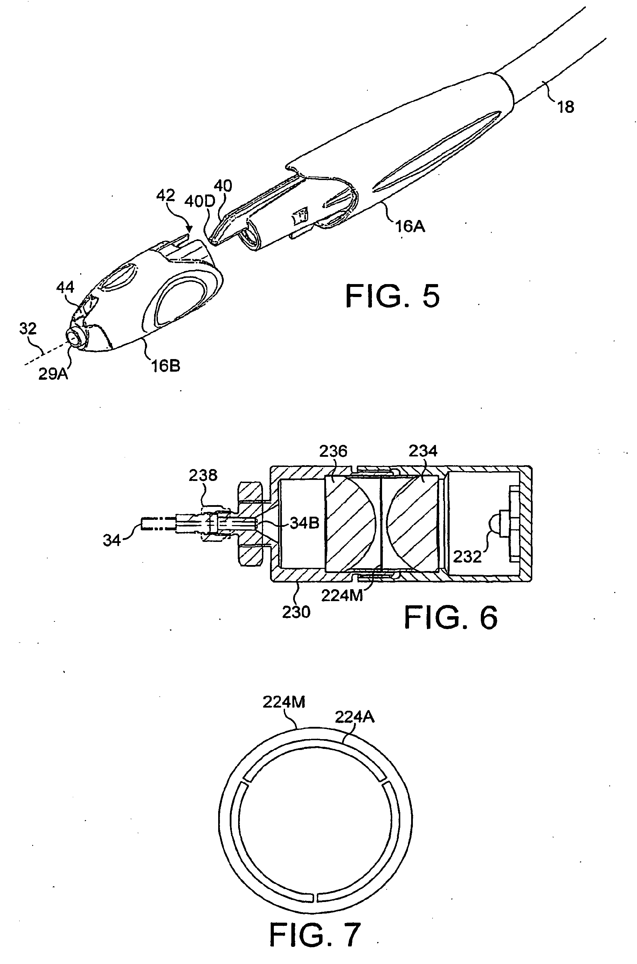

The distal end portion 34A of the optical fibre guide is supported within the disposable nose section 16B by an elongate rigid fibre guide support 40, as shown in the exploded view of the handpiece appearing in Figure 5. When the disposable nose section 16B is fitted to the handpiece body 16A, the fibre guide support 40 extends through a passage 42 in the nose section 16A and is exposed at an aperture 44 of the nose section 16B so that the distal end of the fibre guide 34, which is at the distal end 40Dof the support, lies adjacent the plasma nozzle 29A. The passage 42 in the disposable nose section 16A locates the fibre guide support 40 and, therefore, the distal end portion 34A of the fibre guide, aligning the guide so that it is correctly positioned with respect to the plasma nozzle 29A and the treatment beam axis 32.

Referring to Figure 6, the light source 224 comprises an illuminated mask 224M mounted transversely in an elongate light source housing 230. The mask 224M is illuminated by a light emitting diode (LED) 232 mounted at one end of the housing 230, visible light from the LED 232 passing through a first collimator lens 234, then through the mask 224M, following which it is concentrated by a second lens 236 onto the proximal end 34B of the fibre guide 34 for transmission to the handpiece 16 shown in Figure 4. The fibre guide 34 is removable from the light source housing 230 by releasing an optical fibre connector 238.

The LED 232 is chosen to produce a blue light since this colour has the advantage of being easily seen on a range of skin colours from light to dark. Other colours may, of course, be used.

A laser diode light source may also be used.

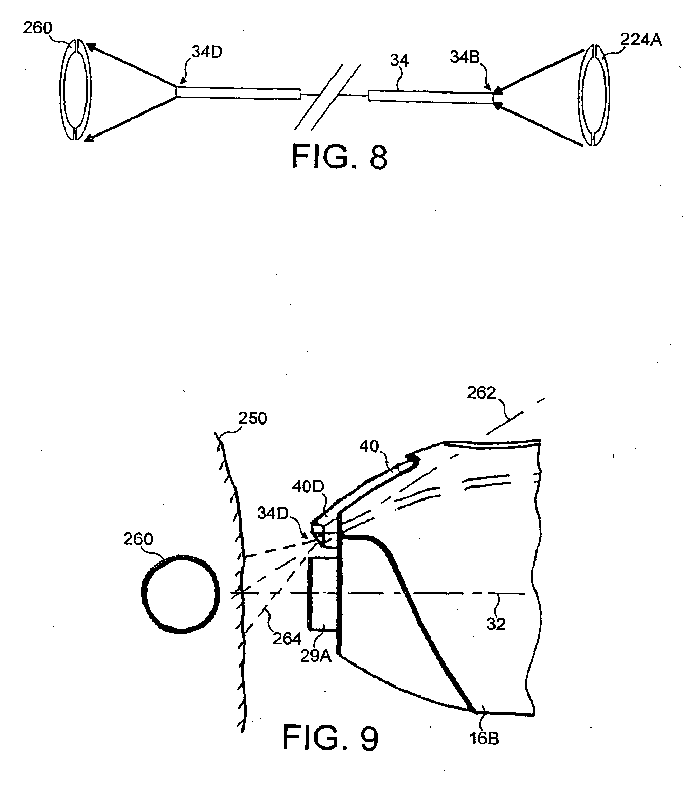

Referring to Figure 7, the light source mask 224M, when viewed in the axial direction of the light source housing 230, is seen to have an annular aperture 224A. It is this aperture 224A which, when illuminated by the LED 232, is imaged on the tissue surface to be treated, albeit with some distortion in the optical fibre guide 34. It is a property of a straight optical fibre with end faces perpendicular to its axis that when light is incident on one of the ends at a given angle to the axis, the light emitted from the other end is emitted at the same angle, providing the angle of incidence is no greater than the so-called "acceptance angle" associated with the material of the fibre. The acceptance angle is sin⁻¹(NA) where NA is the numerical aperture of the fibre. This property of optical fibres, insofar as it relates to the present invention is illustrated in Figure 8. The light from the light source, shown as the illuminated aperture 224A in Figure 8, is focused onto the proximal end 34B of the fibre guide 34, the angle of the edge of the annulus with respect to the fibre axis being less than the acceptance angle for the material of the fibre. At the distal end 34D, light transmitted from the proximal end 34B emerges, as shown, at the same angle with respect to the fibre axis as the incident light at the proximal end, the emerging light then diverging so that an image 260 of the annulus is formed in a plane spaced from the guide distal end 34D. As stated above, light from the light source aperture 224A is concentrated on the guide proximal end 34B by the second lens 236 in the light source housing as described above with reference to Figure 6. In practice, the focal length of this lens is arranged to be greater than the spacing between the lens and the fibre proximal end 34B so that the image of the aperture 224A is spaced beyond the distal end of the fibre guide, as shown in Figure 8.

Multiple internal reflections, the length of the fibre, and bending of the fibre, amongst other effects, tend to spread the incident rays to some degree. The mask 224M is located in a collimated beam of light produced between the two lenses 234, 236 of the light source. Accordingly, with an annular aperture 224A, a cylindrical annulus of light is incident upon the second lens 236. The image of the annulus is transmitted through the fibre guide with a fidelity dependent on the quality of the fibre, its length, and its degree of bending. A low cost polymer fibre may be used. The best results, however, are obtained with a silica fibre, which has lower losses and distortion. Polymer fibres typically have a numerical aperture in the range of from 0.3 to 0.75, while silica fibres have a numerical aperture generally within the range 0.12 to 0.48.

The projection of the annular image 260 onto a target tissue surface will now be described with reference to Figure 9. In its support 40, the distal portion 34A of the fibre guide is bent towards the treatment beam axis 32 so that, at the exit aperture formed by the distal end 34D of the fibre guide, light transmitted through the fibre guide 34 emerges centred in an inclined projection axis 262 which intersects the treatment beam axis 32 at a predetermined spacing from the plasma exit nozzle 29A. The properties of the second lens 236 in the light source housing 230 and of the fibre guide 34 are such that the focused image 260 of the light source annulus 224A appears approximately in a perpendicular plane passing through the intersection of the two axes 32, 262. The degree of divergence of the projected marker beam 264 is such that at the projection plane the size of the image marker 260 is approximately the same as the external diameter of the plasma exit nozzle 29A. The predetermined spacing which is determined by the configuration of the projector, corresponds to the preferred spacing of a tissue surface 250 from the end of the plasma exit nozzle 29A for optimum clinical effect. Accordingly, in use, the correct spacing of the handpiece 16 from the tissue surface 250 can be judged by the user by locating the handpiece so as to produce an image of a required size with reference to the diameter of the exit nozzle 29A. In other words, the size of the marker image 260 indicates the handpiece stand-off distance from the tissue surface as a result of the conical nature projected beam, the axis of the cone being approximately coincident with the exit aperture of the fibre guide. In this embodiment, the handpiece is correctly spaced from the tissue surface when the diameter of the marker is approximately the same as the external diameter of the nozzle. Thus, with a nozzle diameter of 5mm, the instrument may be held so that the marker diameter is about 5mm or 6mm. Thus, with a nozzle diameter of 5mm, the instrument may be held so that the marker diameter is about 5mm or 6mm. The area occupied by the marker 260 also indicates, at least approximately, the area of clinical effect, dependent on the size of the nozzle 29A. In practice the diameter of the treatment energy beam incident on the skin surface at an optimal distance of approximately 5mm from the skin surface is 9mm to 10mm with a corresponding optical marker ring diameter of 6mm. Placing the optical rings adjacent one another during the application of a series of treatment pulses allows for an overlap of the treatment energy such that the effect of Gaussian distribution of energy within the treatment energy beam is eliminated. In other words, a suitable overlap can be achieved by positioning the handpiece so that at each successive location the edge of the marker ring is generally tangential to the annulus illuminated by the marker ring in the previous location.

Large deviations of the treatment beam axis 32 from the preferred perpendicular orientation with respect to the tissue surface 250 are indicated by a pronounced elliptical image (as opposed to a circular or near-circular image).

By actuating the light source before treatment begins, the user can position the handpiece 16 at the required spacing from the tissue surface and can identify the area of clinical effect before the gas plasma is actuated.

Variations to the system include the following.

With appropriate modification to the mask 224M of the light source 224, a solid circle of light may be projected on the tissue surface rather than an annulus.

The exit aperture formed by the distal end 34D of the fibre guide 34 is radially offset with respect to the treatment beam axis, the distal end portion 34A of the fibre guide being bent to project the annulus of light such that the centre of the projected annulus is centrally positioned with respect to the centre of the zone of treatment produced by a gas plasma jet from the nozzle 29A. Alternatively, the distal face of the fibre may be processed such that it is not perpendicular to the fibre axis. In this case, the light is projected at an angle with respect to the fibre axis at its exit aperture and may, thereby, be used to modify the shape of the image and its spacing from the nozzle 29A.

At least one additional fibre guide may be employed between the light source 224 and the distal end of the handpiece. For example, part of the marker image may be transmitted by one fibre guide and another part of the image by another fibre guide. In particular, half of the image may be projected by a fibre guide offset on one side of the plasma exit nozzle and the other half of the image may be projected by a fibre guide terminating on the diametrically opposite side of the nozzle, the respective projection axes intersecting at the required tissue treatment spacing from the nozzle. In this way, the image appears disjointed or mis-shapen at spacings of the instrument greater or less than the optimum spacing.

The quartz tube 29 itself may be used as a light guide for projecting the marker.

It is possible to mount the projector completely within the handset, powering the light source from a battery.

Systems within the broader scope of the invention may include systems in which heating energy is delivered to the tissue from a source having a low thermal time constant. This time constant is preferably less than 500ms, and advantageously less than 200ms. Typically, treatment energy can be delivered in pulses of very short duration (typically 0.5 to 100ms) and without reliance on an intermediary conversion from one kind of energy to another such as a chromophore in laser energy and tissue resistivity in radio frequency energy. In fact, the method of thermal energy transfer to the skin surface, and the elevated temperature of the skin surface produced by the method, have an associated low thermal time constant.

In this connection, the thermal time constant of a material or object is the product of thermal capacitance and thermal resistance, and is a measure of the time taken for the temperature of the material or object to fall to 0.368 of its original temperature when subjected to a step function change in temperature under zero power conditions.

Devices which typically have low thermal time constants are those used for dynamically measuring temperature changes, in which case the thermal time constant is typically of the order of 200ms or less.

When the stored energy of the plasma impacts the skin, the pulse length is typically of the order of 15ms for the transfer of energies of the order of 4 joules, which raises the surface temperature of the skin to approximately 180°C, or 145°C above the ambient temperature. The thermal time constant for the skin/plasma interaction is the time taken for the surface temperature of the skin to fall by 91.6°C. This has been shown to occur in less than 200ms. Once the plasma has given up its energy, it returns to the inert diatomic gas from which it was created, such that the heated skin surface is now exposed to ambient temperature. This is to be compared to an object with a high thermal capacity, such as a hot metallic object, that will extend the thermal time constant. The longer the thermal time constant at the skin surface, the more damage and disruption will occur as cell death is not purely correlated to temperature, but also to the time of exposure to that temperature. The plasma, therefore, has a predictable effect for a given amount of energy. It is, therefore, desirable for the method of applying thermal energy to the skin surface to produce a temperature elevation with a low thermal time constant. Typically, the thermal time constant should be less than 500mS, and preferably less than 200mS.

In use; the instrument 16 is passed over the surface of tissue to be cosmetically treated, with the nozzle 29a typically being held a few millimetres, e.g. about 5 millimetres, from the surface of the tissue. The pulse duration and energy levels are chosen so as to form first and second adjacent regions of thermally-modified tissue in the region of the DE Junction. The first, upper region is termed a zone of thermal damage, having a thermal modification which is greater than that of the second, lower region. The thermally damaged zone is thermally modified to an extent that it separates from the second region some days after the delivery of the thermal energy. Following separation of the first damaged region, the epidermis and the upper region of the dermis regenerate naturally.

A benefit of using a diatomic plasma is that it is able to deliver a relatively large amount of energy which causes heating in a short period of time. This enables delivery in discreet pulses of millisecond duration, and is in contrast to heat conduction from a merely hot gas. In the preferred embodiment, energy from 1 Joule to 4 Joules is delivered in a period of 4.5 to 15.4 milliseconds respectively for a nozzle with an exit diameter of 5 millimetres, and delivers from less than 0.5 Joules up to 2 Joules in the same period for a nozzle with an exit diameter of less than 1.5 millimetres. Experiments have shown that useful clinical effects are achieved with yet longer pulses extending to 50 milliseconds, and further analysis shows extension up to 100 milliseconds or more will provide useful effects. In addition, the pulse width may be shortened to deliver the same, or otherwise similar, useful heating energy. Plasma pulses as short as 0.5 milliseconds have been produced with the system described above.

Another benefit is that oxygen is purged from the skin surface by the plasma and flow of inert gas that follows immediately following a plasma pulse. As a result, the oxidative carbonisation that often occurs at the skin surface on application of thermal energy is avoided, leaving a desiccated intact epithelium with minor structural alteration.

This minor structural alteration is nonetheless important in providing yet another benefit of the invention, as it changes the thermal characteristics of the epidermis at higher energy settings. Following a single pass of plasma over the skin surface at an energy setting greater than 2 Joules, the epidermal cells at the basal membrane are heated to a degree that produces vacuolation of the cellular contents. This produces a natural insulator limiting the absorption and depth of penetration of energy from subsequent passes. This is a beneficial safety feature that avoids the risk of excessive damage by inadvertent application of multiple passes to the same site on the skin surface.

Alternatively, when using energy pulses at or below 2 Joules, then the vacuolation is not observed, and the treated skin is still capable of absorbing the thermal energy of a second pass, by changing the energy in the second pass using either a narrow nozzle to focus the plasma or a higher energy setting will have an additive effect. The benefit of using a narrow nozzle embodiment is that the focused energy can be directed onto specific areas of the skin surface such as deeper wrinkles.

For example, if the skin is subjected to two passes of 4 Joules, then the depth of thermal effect is only 10-20% greater than with a single pass of 4 Joules. Alternatively, if the skin is first treated with 2 Joules, then with a second pass of 4 Joules then the effect will be consistent with a single pass with 6 Joules. Part of this benefit also relates to the water content of the skin, particularly the upper layers of the epidermis following pre-treatment with a topical anaesthetic.

Through experimentation with the invention, it has become clear that the depth of effect changes by up to 50% depending on the hydration of the upper layers of the epidermis following application of a topical anaesthetic. Topical anaesthetics include a hydrating component, as they rely on hydration of the superficial epidermis for the penetration of the anaesthetic agent through the skin. This changes the absorption of pure thermal energy, whereby a larger proportion of the energy is dissipated in the superficial epidermis, reducing the depth of penetration into the dermis. If no anaesthesia or tumescent subcuticular anaesthesia is employed, then the depth of dermal penetration for a given energy setting can be doubled. A pre-treatment with 2 Joules produces sufficient desiccation of the superficial epidermis, following use of topical anaesthesia, that an equivalent depth of effect can be produced with the second pass to that achieved with no anaesthesia or tumescent subcuticular anaesthesia.

The reason for using a diatomic plasma which delivers a relatively large amount of energy in a short period of time is that the irreversible clinical effects (the thermal modification and thermal damage of the tissue) occur over tissue depths that result in the desired clinical effects, whilst avoiding any undesired clinical effects. If the heating energy is delivered over too long a time, the effects of convection from the skins surface and conduction into the underlying tissue will be such that no significant temperature rise results. On the other hand, if the time is too short, then irreversible effects (such as water vaporising) at or near the skins surface will carry away otherwise useful heating energy.

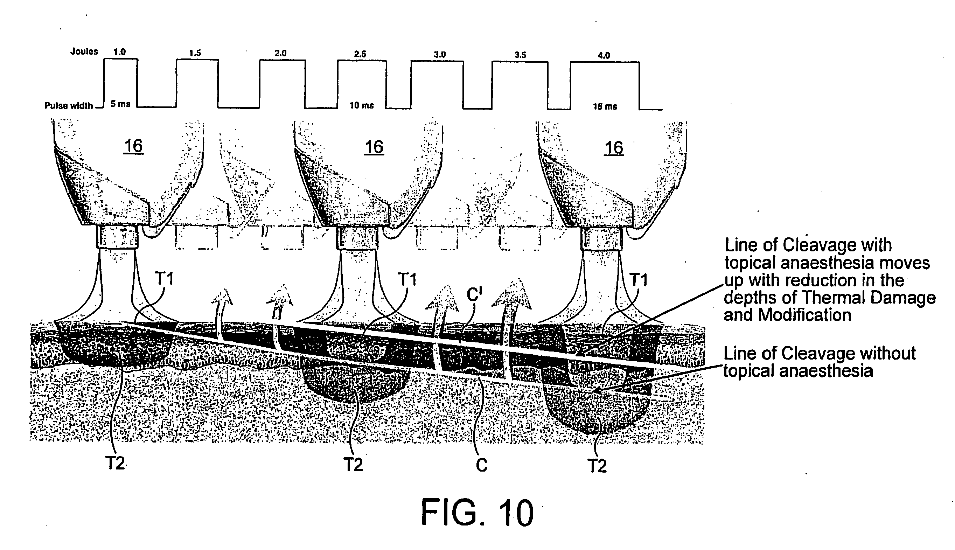

Figure 10 shows the regeneration of the reticular architecture of the dermis for different pulse widths and energy ratings, and illustrates the use of a thermal source with a low thermal time constant. Thus, for an energy setting of 1 Joule, a pulse width of about 4.5 or 5 milliseconds is appropriate, for an energy setting of 2.5 Joules, a pulse width of 10 milliseconds is appropriate, and for an energy setting of 4 Joules, a pulse width of about 15 milliseconds is appropriate. Figure 10 also shows the two regions of thermal modification T1 and T2, T1 being the upper region of thermal damage, and T2 being the lower region of thermal modification. Figure 10 also shows the line of cleavage C which develops between these two regions between two and five days after treatment. As is apparent, the depth of effect increases as the energy level and pulse width used for the treatment increases. The dermatologist carrying out the procedure will, therefore, choose the appropriate energy level and pulse width depending on the depth of effect required.

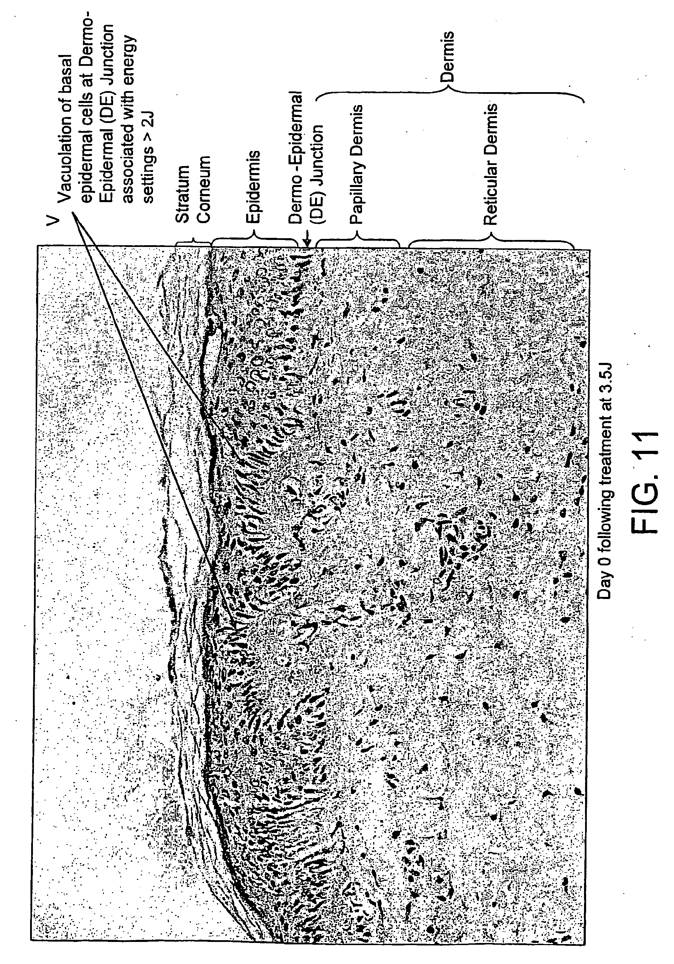

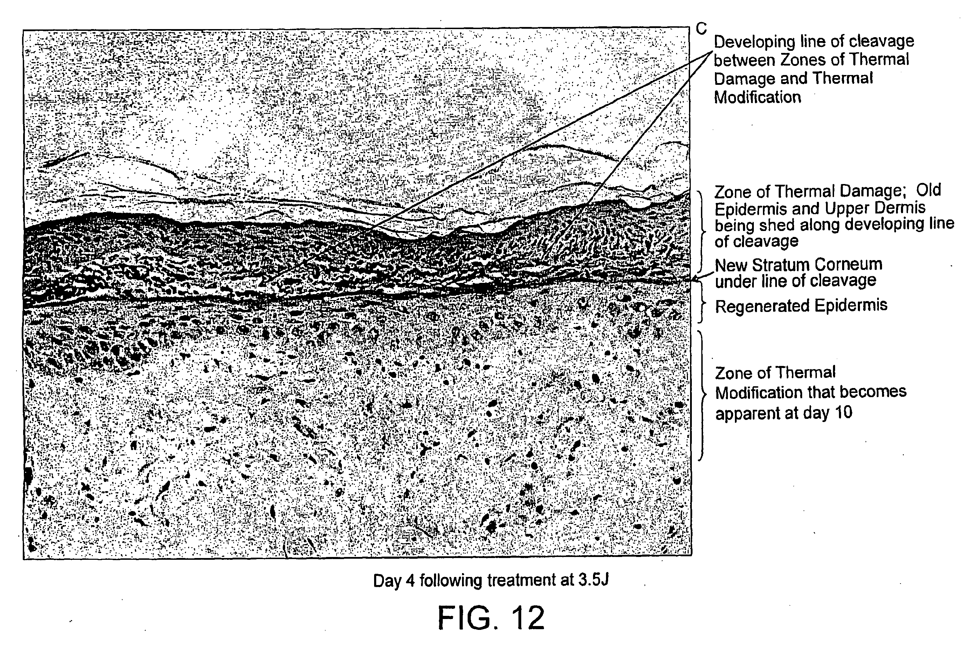

As mentioned above, the use of a topical anaesthetic modifies the effect of the treatment. Thus, as shown in Figure 10 the line of cleavage C is for treatment without a topical anaesthetic, the equivalent line of cleavage (C1) being higher, owing to a reduction in the depth of thermal damage and modification which results from pre-treatment with a topical anaesthetic. Figures 11 to 13 show a typical treatment, and the progress of regeneration of the reticular architecture after the treatment. Thus, Figure 11 shows the effect of treatment at 3.5 Joules and a pulse width of 13.6 milliseconds immediately following treatment. The Figure shows the dermis (including the reticular dermis and the papillary dermis), the DE Junction, the epidermis and the stratum corneum. Vacuolation of basal epidermal cells at the DE Junction is clearly visible, as indicated by the reference V. Figure 12 shows the position at day four following treatment at 3.5 Joules, and shows a developing line of cleavage C between the regions T1 and T2 of thermal damage and thermal modification. The region T1 of thermal damage is the old epidermis and the upper dermis, which is in the process of being shed along the developing line of cleavage C. Underneath the line of cleavage C a new stratum corneum and a regenerated epidermis are being developed naturally. Figure 12 also shows the zone where thermal modification will later become apparent.

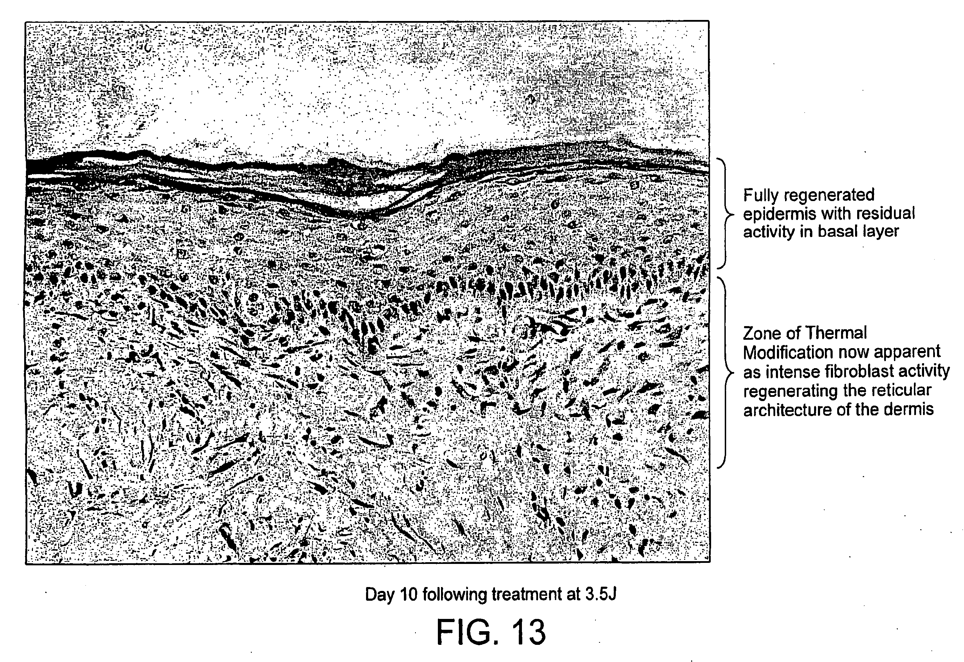

Figure 13 shows the position at day ten following treatment at 3.5 Joules. Here, the epidermis has been fully regenerated with residual activity in the basal layer, and the zone of thermal modification is now apparent, as intense fibroblast activity regenerates the reticular architecture of the dermis.

Referring again to Figure 10, it will be observed that, in this case, the instrument is positioned perpendicularly with respect to the tissue surface. A reduction in the clinical effect for a given energy output can be achieved by inclining the handpiece 16 with respect to the tissue surface or by increasing the spacing between the handpiece 16 and the tissue surface. Such techniques are particularly useful for blending the effect between a fully treated area of the skin and an untreated area. This can be seen as a "feathering" technique. The target marker may be used here, also, as an instrument positioning aid. As the instrument is moved outwardly towards the edge of the area to be treated, its position may be progressively changed, e.g., with increasing inclination with respect to the tissue surface, as happens when the instrument is moved so as to project a marker of increasingly elliptical shape. Alternatively or in addition, the instrument may be moved so that the marker increases in size with outward movement towards the boundary of the area of treatment.

## Claims

1. A tissue treatment system including a treatment instrument in the form of a handpiece which is arranged to direct a beam of treatment energy from a distal end of the handpiece along a treatment beam axis for treating a tissue surface spaced from the said distal end, the treatment energy being produced by a treatment energy emitter, wherein the handpiece incorporates at least part of an optical target marking projector for projecting a visible target marker, wherein the target marking projector has a light exit aperture at the distal end of the handpiece, the light exit aperture being offset from the treatment beam axis, and wherein the projector has a projection axis which is inclined towards the treatment beam axis to intersect the latter substantially at a predetermined spacing from the handpiece distal end, the projector being arranged to project the marker onto a plane at the predetermined spacing.

2. A system according to claim 1, wherein the treatment energy emitter is a gas plasma generator and the handpiece has a nozzle at its distal end for directing an energy beam in the form of a plasma jet outwardly from the nozzle.

3. A system according to claim 1 or claim 2, wherein the light exit aperture is in the region of a distal end of the handpiece and is arranged to project a diverging light beam from the exit aperture in order that, in use of the system, the size of the projected marker on the tissue surface varies according to the spacing of the handpiece from the tissue surface.

4. A system according to claim 3, arranged to emit a substantially conical beam.

5. A system according to claim 3 or claim 4, wherein the projector comprises a light source and an optical fibre light guide having an entrance end and an exit end, at least a distal portion of the light guide being housed in the handpiece, and wherein the light source is arranged such that at least a component of light generated by the light source and incident on the entrance end of the light guide is incident at an angle to a central axis of light guide.

6. A system according to claim 5, wherein the light source is arranged to generate a converging light beam which converges on the said entrance end of the light guide.

7. A system according to any preceding claim, wherein the target marking projector is arranged to emit a diverging light beam the lateral extent of which provides a defined reference to the lateral extent of the treatment energy beam at at least one predetermined distance from the handpiece distal end.

8. A system according to any preceding claim, arranged to direct the treatment energy beam along a treatment beam axis, wherein the target marking projector is configured to project a target marker which is substantially circular in a plane perpendicular to the treatment axis.

9. A system according to claim 1, wherein the target marking projector has a light exit aperture at the distal end of the handpiece.

10. A system according to claim 1 or claim 9, wherein the projected target marker defines an indicated area in a projection plane at the said predetermined spacing, which area is indicative of the tissue area treated which will be treated by the treatment energy beam.

11. A system according to claim 10, wherein the target marker comprises a marker ring.

12. A system according to any preceding claim, wherein the projector comprises a light source and an optical fibre light guide, at least a distal portion of which is housed in the handpiece and which terminates in the exit aperture.

13. A system according to claim 12, wherein the light source is formed in the shape of the required marker and the projector includes a lens for concentrating light from the light source onto a proximal end of the fibre guide.

14. A system according to claim 13, wherein the light source is an illuminated mask, the mask having a marker aperture formed in the shape of the marker.

15. A system according to claim 14, wherein the mask is illuminated by a diode source and is located in a collimator.

16. A system according to claim 14, wherein the marker aperture is annular.

17. A system according to any of claims 12 to 16, including a treatment energy power source in a power source housing, the handpiece being connected to the power source by a cord for supplying treatment energy power to the handpiece, where the light source is in the power source housing and the optical fibre guide extends through the cord into the handpiece.

18. A system according to any of claims 12 to 17, wherein the handpiece has a disposable nose section and a re-usable body, and wherein the handpiece body has a distally extending substantially rigid fibre guide support which houses the distal portion of the fibre and extends through a passage in the disposable nose section.

19. A system according to any of claims 12 to 18, wherein the distal portion of the fibre guide is bent in the handpiece towards a treatment beam axis of the handpiece so as to define an inclined projection axis at the distal end of the fibre guide.

20. A system according to claim 19, wherein the distal end of the fibre guide has a distal face which is perpendicular to the projection axis.

21. A tissue treatment instrument for a tissue treatment system, wherein the instrument comprises a handpiece which is arranged to direct a beam of treatment energy from a distal end thereof along a treatment beam axis defined by the handpiece for treating a tissue surface spaced from the said distal end, wherein the handpiece incorporates optical means for projecting a visible target marker, and wherein the optical means has a light exit aperture which is offset from the treatment beam axis and
defines a projection axis inclined with respect to the treatment beam axis to intersect the latter substantially at a predetermined spacing from the handpiece distal end, the optical means being arranged to project the marker onto a plane at the predetermined spacing.

22. An instrument according to claim 21, wherein the handpiece has a nozzle at its distal end for directing the treatment energy beam along a treatment beam axis and houses an optical fibre light guide, the light exit aperture forming part of the light guide and being located adjacent the nozzle.

23. An instrument according to claim 22, including a light source for directing light onto an entrance end of the light guide, the light guide and the source being arranged so as to project a diverging light beam from the light exit aperture.

24. An instrument according to claim 23, wherein the beam is such that the target marker is substantially circular in a plane extending transversely with respect to the treatment beam axis.

25. An instrument according to claim 21, including a gas plasma generator having a nozzle at the distal end of the handpiece for directing an energy beam in the form of a plasma jet outwardly from the nozzle, wherein the said optical means comprises an optical light guide which terminates adjacent the nozzle.

## Patentansprüche

1. Gewebebehandlungssystem, das ein Behandlungsinstrument in Form eines Handgeräts umfasst, welches dafür ausgelegt ist, einen Behandlungsenergiestrahl von einem distalen Ende des Handgeräts aus entlang einer Behandlungsstrahlachse zu richten, um eine Gewebeoberfläche zu behandeln, die von dem distalen Ende beabstandet ist, wobei die Behandlungsenergie von einem Behandlungsenergieemitter erzeugt wird, wobei in das Handgerät zumindest ein Teil eines optischen Zielmarkierungsprojektors zum Projizieren einer sichtbaren Zielmarkierung integriert ist, wobei der Zielmarkierungsprojektor eine Lichtaustrittsöffnung an dem distalen Ende des Handgeräts aufweist, wobei die Lichtaustrittsöffnung zu der Behandlungsstrahlachse versetzt ist und wobei der Projektor eine Projektionsachse aufweist, die zu der Behandlungsstrahlachse geneigt ist, so dass sie letztere im Wesentlichen in einem vorgegebenen Abstand von dem distalen Ende des Handgeräts schneidet, wobei der Projektor dafür ausgelegt ist, die Markierung in dem vorgegebenen Abstand auf eine Ebene zu projizieren.

2. System nach Anspruch 1, bei welchem der Behandlungsenergieemitter ein Gasplasmagenerator ist und das Handgerät eine Düse an seinem distalen Ende aufweist, um einen Energiestrahl in Form eines Plasmastrahls von der Düse nach außen zu richten.

3. System nach Anspruch 1 oder Anspruch 2, bei welchem sich die Lichtaustrittsöffnung im Bereich eines distalen Endes des Handgeräts befindet und dafür ausgelegt ist, von der Austrittsöffnung aus einen divergierenden Lichtstrahl zu projizieren, so dass sich beim Einsatz des Systems die Größe der projizierten Markierung auf der Gewebeoberfläche entsprechend dem Abstand des Handgeräts von der Gewebeoberfläche ändert.

4. System nach Anspruch 3, welches dafür ausgelegt ist, einen im Wesentlichen konischen Strahl zu emittieren.

5. System nach Anspruch 3 oder Anspruch 4, bei welchem der Projektor eine Lichtquelle sowie eine faseroptische Lichtleitung mit einem Eintrittsende und einem Austrittsende umfasst, wobei zumindest ein distaler Abschnitt der Lichtleitung in dem Handgerät aufgenommen ist und wobei die Lichtquelle derart ausgelegt ist, dass zumindest eine Komponente des Lichts, das von der Lichtquelle erzeugt wird und auf das Eintrittsende der Lichtleitung auftrifft, in einem Winkel zu einer Mittelachse der Lichtleitung auftrifft.

6. System nach Anspruch 5, bei welchem die Lichtquelle derart ausgelegt ist, dass sie einen konvergierenden Lichtstrahl erzeugt, welcher an dem Eintrittsende der Lichtleitung konvergiert.

7. System nach einem der vorhergehenden Ansprüche, bei welchem der Zielmarkierungsprojektor dafür ausgelegt ist, einen divergierenden Lichtstrahl zu emittieren, dessen seitliche Ausdehnung eine definierte Bezugsgröße für die seitliche Ausdehnung des Behandlungsenergiestrahls in zumindest einem vorgegebenen Abstand von dem distalen Ende des Handgeräts bereitstellt.

8. System nach einem der vorhergehenden Ansprüche, welches dafür ausgelegt ist, den Behandlungsenergiestrahl entlang einer Behandlungsstrahlachse zu richten, wobei der Zielmarkierungsprojektor dafür konfiguriert ist, eine Zielmarkierung zu projizieren, die in einer Ebene senkrecht zu der Behandlungsachse im Wesentlichen kreisförmig ist.

9. System nach Anspruch 1, bei welchem der Zielmarkierungsprojektor an dem distalen Ende des Handgeräts eine Lichtaustrittsöffnung aufweist.

10. System nach Anspruch 1 oder Anspruch 9, bei welchem die projizierte Zielmarkierung einen angezeigten Bereich in einer Projektionsebene in dem vorgegebenen Abstand definiert, wobei der Bereich den behandelten Gewebebereich angibt, der durch den Behandlungsenergiestrahl behandelt wird.

11. System nach Anspruch 10, bei welchem die Zielmarkierung einen Markierungsring umfasst.

12. System nach einem der vorhergehenden Ansprüche, bei welchem der Projektor eine Lichtquelle sowie eine faseroptische Lichtleitung umfasst, wobei zumindest ein distaler Abschnitt derselben in dem Handgerät aufgenommen ist und in der Austrittsöffnung endet.

13. System nach Anspruch 12, bei welchem die Lichtquelle in Gestalt der erforderlichen Markierung geformt ist und der Projektor eine Linse zum Konzentrieren des Lichts von der Lichtquelle auf ein proximales Ende der Faserleitung umfasst.

14. System nach Anspruch 13, bei welchem die Lichtquelle eine beleuchtete Maske darstellt, wobei die Maske eine Markierungsöffnung aufweist, die in Gestalt der Markierung geformt ist.

15. System nach Anspruch 14, bei welchem die Maske durch eine Diodenquelle beleuchtet wird und in einem Kollimator angeordnet ist.

16. System nach Anspruch 14, bei welchem die Markierungsöffnung ringförmig ist.

17. System nach einem der Ansprüche 12 bis 16, welches eine Behandlungsenergie-Leistungsquelle in einem Leistungsquellengehäuse umfasst, wobei das Handgerät durch ein Kabel mit der Leistungsquelle verbunden ist, um Behandlungsenergieleistung an das Handgerät zu liefern, wobei sich die Lichtquelle in dem Leistungsquellengehäuse befindet und die faseroptische Leitung sich durch das Kabel hindurch in das Handgerät erstreckt.

18. System nach einem der Ansprüche 12 bis 17, bei welchem das Handgerät einen Einweg-Ansatzteil sowie einen wiederverwendbaren Korpus aufweist und bei welchem der Handgerätkorpus eine sich in distaler Richtung erstreckende, im Wesentlichen starre Faserleitungshalterung aufweist, welche den distalen Abschnitt der Faser aufnimmt und sich durch einen Kanal in dem Einweg-Ansatzteil hindurch erstreckt.

19. System nach einem der Ansprüche 12 bis 18, bei welchem der distale Abschnitt der Faserleitung in dem Handgerät zu einer Behandlungsstrahlachse des Handgeräts hin gebogen ist, so dass er an dem distalen Ende der Faserleitung eine schräge Projektionsachse definiert.

20. System nach Anspruch 19, bei welchem das distale Ende der Faserleitung eine distale Fläche aufweist, die senkrecht zu der Projektionsachse liegt.

21. Gewebebehandlungsinstrument für ein Gewebebehandlungssystem, wobei das Instrument ein Handgerät umfasst, das dafür ausgelegt ist, von seinem distalen Ende aus einen Behandlungsenergiestrahl entlang einer Behandlungsstrahlachse zu richten, die durch das Handgerät definiert wird, um eine Gewebeoberfläche zu behandeln, die von dem distalen Ende beabstandet ist, wobei das Handgerät optische Mittel zum Projizieren einer sichtbaren Zielmarkierung umfasst und wobei die optischen Mittel eine Lichtaustrittsöffnung aufweisen, die zu der Behandlungsstrahlachse versetzt ist und eine Projektionsachse definiert, die in Bezug auf die Behandlungsstrahlachse geneigt ist, so dass sie letztere im Wesentlichen in einem vorgegebenen Abstand von dem distalen Ende des Handgeräts aus schneidet, wobei die optischen Mittel derart angeordnet sind, dass sie die Markierung in dem vorgegebenen Abstand auf eine Ebene projizieren.

22. Instrument nach Anspruch 21, wobei das Handgerät an seinem distalen Ende eine Düse aufweist, um den Behandlungsenergiestrahl entlang einer Behandlungsstrahlachse zu richten, wobei in diesem eine faseroptische Lichtleitung aufgenommen ist, wobei die Lichtaustrittsöffnung einen Teil der Lichtleitung bildet und angrenzend an die Düse angeordnet ist.

23. Instrument nach Anspruch 22, welches eine Lichtquelle zum Richten von Licht auf ein Eintrittsende der Lichtleitung umfasst, wobei die Lichtleitung und die Quelle derart angeordnet sind, dass von der Lichtaustrittsöffnung aus ein divergierender Lichtstrahl projiziert wird.

24. Instrument nach Anspruch 23, bei welchem der Strahl derart beschaffen ist, dass die Zielmarkierung in einer Ebene, die sich senkrecht in Bezug auf die Behandlungsstrahlachse erstreckt, im Wesentlichen kreisförmig ist.

25. Instrument nach Anspruch 21, welches einen Gasplasmagenerator mit einer Düse an dem distalen Ende des Handgeräts aufweist, um einen Energiestrahl in Form eines Plasmastrahls von der Düse nach außen zu richten, wobei die optischen Mittel eine optische Lichtleitung umfassen, die angrenzend an die Düse endet.

## Revendications

1. Système de traitement de tissu comportant un instrument de traitement sous forme d'une pièce à main qui est agencée pour diriger un faisceau d'énergie de traitement depuis une extrémité distale de la pièce à main le long d'un axe de faisceau de traitement pour traiter une surface de tissu espacée de ladite extrémité distale, l'énergie de traitement étant produite par un émetteur d'énergie de traitement, dans lequel la pièce à main incorpore au moins une partie d'un projecteur de marquage de cible optique pour projeter un marqueur de cible visible, dans lequel le projecteur de marquage de cible présente une ouverture de sortie de lumière à l'extrémité distale de la pièce à main, l'ouverture de sortie de lumière étant décalée de l'axe du faisceau de traitement, et dans lequel le projecteur a un axe de projection qui est incliné vers l'axe du faisceau de traitement pour couper celui-ci sensiblement à un espacement prédéterminé depuis l'extrémité distale de la pièce à main, le projecteur étant agencé pour projeter le marqueur sur un plan à l'espacement prédéterminé.

2. Système selon la revendication 1, dans lequel l'émetteur d'énergie de traitement est un générateur à plasma gazeux et la pièce à main présente à son extrémité distale une buse pour diriger un faisceau d'énergie sous forme d'un jet de plasma vers l'extérieur à partir de la buse.

3. Système selon la revendication 1 ou la revendication 2, dans lequel l'ouverture de sortie de lumière est dans la région de l'extrémité distale de la pièce à main et est agencée pour projeter un faisceau divergent de lumière à partir de l'ouverture de sortie de façon que, quand on utilise le système, la taille du marqueur projeté sur la surface du tissu varie en fonction de l'espacement de la pièce à main depuis la surface du tissu.

4. Système selon la revendication 3, agencé pour émettre un faisceau sensiblement conique.

5. Système selon la revendication 3 ou la revendication 4, dans lequel le projecteur comprend une source de lumière et un guide de lumière à fibre optique ayant une extrémité d'entrée et une extrémité de sortie, au moins une partie du guide de lumière étant logée dans la pièce à main, et dans lequel la source de lumière est agencée de manière qu'au moins une composante de lumière générée par la source de lumière et incidente à l'extrémité d'entrée du guide de lumière soit incidente en formant un angle avec l'axe central du guide de lumière.

6. Système selon la revendication 5, dans lequel la source de lumière est agencée pour générer un faisceau convergent de lumière qui converge sur ladite extrémité d'entrée du guide de lumière.

7. Système selon l'une quelconque des revendications précédentes, dans lequel le projecteur de marquage de cible est agencé pour émettre un faisceau divergent de lumière dont l'étendue latérale fournit une référence définie à l'étendue latérale du faisceau d'énergie de traitement à au moins une distance prédéterminée de l'extrémité distale de la pièce à main.

8. Système selon l'une quelconque des revendications précédentes, agencé pour diriger le faisceau d'énergie de traitement le long d'un axe de faisceau de traitement, dans lequel le projecteur de marquage de cible est configuré pour projeter un marqueur de cible qui est sensiblement circulaire dans un plan perpendiculaire à l'axe de traitement.

9. Système selon la revendication 1, dans lequel le projecteur de marquage de cible présente une ouverture de sortie de lumière à l'extrémité distale de la pièce à main.

10. Système selon la revendication 1 ou la revendication 9, dans lequel le marqueur de cible projetée définit une zone indiquée dans un plan de projection audit espacement prédéterminé, laquelle zone est indicative d'une zone de tissu traitée qui sera traitée par le faisceau d'énergie de traitement.

11. Système selon la revendication 10, dans lequel le marqueur de cible comprend un anneau marqueur.

12. Système selon l'une quelconque des revendications précédentes, dans lequel le projecteur comprend une source de lumière et un guide de lumière à fibre optique, dont au moins une partie distale est logée dans la pièce à main et qui se termine dans l'ouverture de sortie.

13. Système selon la revendication 12, dans lequel la source de lumière est conformée à la forme du marqueur requis et le projecteur comporte une lentille pour concentrer la lumière depuis la source de lumière sur une extrémité proximale du guide à fibre.

14. Système selon la revendication 13, dans lequel la source de lumière est un masque illuminé, le masque présentant une ouverture de marqueur conformée à la forme du marqueur.

15. Système selon la revendication 14, dans lequel le masque est illuminé par une source à diode et est placé dans un collimateur.

16. Système selon la revendication 14, dans lequel l'ouverture de marqueur est annulaire.

17. Système selon l'une quelconque des revendications 12 à 16, comportant une source de puissance d'énergie de traitement dans un logement de source de puissance, la pièce à main étant reliée à la source de puissance par un cordon pour fournir la puissance d'énergie de traitement à la pièce à main, la source lumineuse étant dans le logement de la source de puissance et le guide à fibre optique s'étendant par l'intermédiaire du cordon dans la pièce à main.

18. Système selon l'une quelconque des revendications 12 à 17, dans lequel la pièce à main à une section de bec à l'avant jetable et un corps réutilisable, et dans lequel le corps de la pièce à main présente un support de guide à fibre sensiblement rigide et s'étendant de manière distale qui loge la parte distale de la fibre et s'étend à travers un passage dans la section de bec jetable.

19. Système selon l'une quelconque des revendications 12 à 18, dans lequel la partie distale du guide à fibre est courbée dans la pièce à main vers l'axe du faisceau de traitement de la pièce à main de manière à définir un axe de projection incliné à l'extrémité distale du guide à fibre.

20. Système selon la revendication 19, dans lequel l'extrémité distale du guide à fibre présente une face distale qui est perpendiculaire à l'axe de projection.

21. Instrument de traitement de tissu pour un système de traitement de tissu, dans lequel l'instrument comporte une pièce à main qui est agencée pour diriger un faisceau d'énergie de traitement depuis une extrémité distale de la pièce à main le long d'un axe de faisceau de traitement défini par la pièce à main pour traiter une surface de tissu espacée de ladite extrémité distale, dans lequel la pièce à main incorpore des moyens optiques pour projeter un marqueur de cible visible, dans lequel les moyens optiques présentent une ouverture de sortie de lumière décalée de l'axe du faisceau de traitement et définissent un axe de projection qui est incliné par rapport à l'axe du faisceau de traitement pour couper celui-ci sensiblement à un espacement prédéterminé depuis l'extrémité distale de la pièce à main, les moyens optiques étant agencés pour projeter le marqueur sur un plan à l'espacement prédéterminé.

22. Système selon la revendication 21, dans lequel la pièce à main présente à son extrémité distale une buse pour diriger un faisceau d'énergie de traitement le long d'un axe de faisceau de traitement et loge un guide de lumière à fibre optique, l'ouverture de sortie de lumière formant une partie du guide de lumière et étant située adjacente à la buse.

23. Système selon la revendication 22, comportant une source de lumière pour diriger la lumière sur une extrémité d'entrée du guide de lumière, le guide de lumière et la source étant disposées de manière à projeter un rayon de lumière divergente à partir de l'ouverture de sortie de lumière.

24. Système selon la revendication 23, dans lequel le faisceau est tel que le marqueur de cible soit sensiblement circulaire dans un plan s'étendant perpendiculairement à l'axe du faisceau de traitement.

25. Système selon la revendication 21, comportant un générateur à plasma gazeux présentant à l'extrémité distale de la pièce à main une buse pour diriger un faisceau d'énergie sous forme d'un jet de plasma vers l'extérieur à partir de la buse, dans lequel lesdits moyens optiques comprennent un guide de lumière optique qui se termine de manière adjacent à la buse.
